# EUROPÄISCHE PATENTSCHRIFT

(11) **EP 1 230 229 B1**
(45) Veröffentlichungstag und Bekanntmachung des Hinweises auf die Patenterteilung: **28.05.2003**
(21) Anmeldenummer: 00967893.9
(22) Anmeldetag: 20.10.2000
(51) Int. Cl.: C07D 277/32, C07D 277/40, C07C 331/20

(54) **VERFAHREN ZUR HERSTELLUNG VON 2-CHLOR-5-CHLORMETHYL-1,3-THIAZOL**
METHOD FOR PRODUCING 2-CHLORO-5-CHLOROMETHYL-1,3-THIAZOL
PROCEDE DE PRODUCTION DE 2-CHLORO-5-CHLOROMETHYL-1,3-THIAZOL

(30) Priorität: 15.11.1999 AT 191499; 14.01.2000 AT 462000
(43) Veröffentlichungstag der Anmeldung: 14.08.2002
(73) Patentinhaber: DSM Fine Chemicals Austria Nfg GmbH & Co KG, 4021 Linz (AT)
(72) Erfinder: HENDEL, Wolfram, A-4060 Leonding (AT); KRICH, Silvia, A-4203 Altenberg/Linz (AT)
(74) Vertreter: Lindinger, Ingrid
(86) Internationale Anmeldenummer: EP0010318
(87) Internationale Veröffentlichungsnummer: WO01036400

(56) Entgegenhaltungen:
- EP-A- 0 446 913
- EP-A- 0 763 531
- EP-A- 0 780 384
- EP-A- 0 794 180
- GOEBEL T ET AL: "Synthetic approaches towards CGA 293'343: a novel broad-spectrum insecticide" PESTICIDE SCIENCE, Bd. 55, Nr. 3, 1999, Seiten 355-357, XP000960525 ISSN: 0031-613X

## Beschreibung

Die Erfindung betrifft Verfahren zur Herstellung von 2-Chlor-5-chlormethyl-1,3-thiazol (CCT), sowie darin verwendete Zwischenverbindungen.

2-Chlor-5-chlormethyl-1,3-thiazol ist ein wertvolles Zwischenprodukt bei der Herstellung von Pestiziden oder von pharmazeutischen Produkten.

Aus der Literatur ist bereits eine Vielzahl der unterschiedlichsten Verfahren zur Herstellung von CCT bekannt.
So wird beispielsweise in EP 0 260 560 und in EP 0 446 913 die Herstellung von CCT durch Reaktion von Allylisothiocyanat bzw. von mit einer Abgangsgruppe substituierten Allylisothiocyanat mit einem Chlorierungsmittel und in EP 0 763 531 die Umsetzung von 2-Chlorallylisothiocyanat mit einem Chlorierungsmittel beschrieben. Diese Verfahren weisen Nachteile auf, da z.B. bei der ersten Variante mehrere Nebenprodukte auftreten, wodurch das hergestellte CCT eine geringe Reinheit aufweist, und das Ausgangsmaterial bei der zweiten Variante nur zu hohen Kosten erhältlich ist. Weiters muss ein beträchtlicher Überschuss an Chlorierungsmittel verwendet werden und es muss in großer Verdünnung gearbeitet werden. Ebenso ist ein exaktes Einhalten der Reaktionstemperatur erforderlich und die sich im Reaktionsverlauf bildende stabile Zwischenstufe muss in einem zusätzlichen Reaktionsschritt exotherm in das gewünschte Endprodukt überführt werden. Als Verbesserung wird in EP 0 794 180 die Herstellung von CCT aus 1,3-Dichlorpropen und einem Thiocyanatsalz über 3-Chlor-1-isothiocyanat-1-propen beschrieben.
Anderer Varianten, wie etwa das Verfahren gemäß EP 0 775 700, wonach CCT über 2-Amino-5-methylthiazol mittels Diazotierung und anschließender Chlorierung hergestellt wird, zeigen ebenfalls den Nachteil, dass CCT durch eine Vielzahl von Nebenprodukten verunreinigt ist, die kaum bzw. nur sehr schwierig und unter hohen Ausbeuteverlusten abzutrennen sind.

Aufgabe der Erfindung war es neue Verfahren zu finden, die die Herstellung von CCT in hoher Reinheit und Ausbeute ermöglichen.

Gegenstand der Erfindung ist demnach ein Verfahren zur Herstellung von 2-Chlor-5-chlormethyl-1,3-thiazol, das dadurch gekennzeichnet ist, dass eine Verbindung der Formel 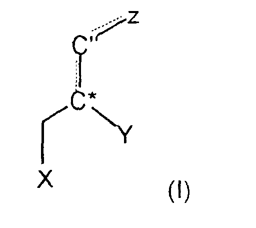 in der X Cl, -OR, -SR oder NR₂ bedeutet, wobei R H oder eine geeignete Schutzgruppe ist; Y H oder Cl und Z Cl oder O bedeuten,
wobei die Verbindungen der Formel (I) maximal eine Doppelbindung zwischen C* und C" oder zwischen C" und Z aufweist, mit der Maßgabe, dass die Bindung zwischen C" und Z, wenn Z gleich O ist, eine Doppelbindung und wenn Z gleich Cl ist, eine Einfachbindung
ist,
a) falls X und Y gleich Cl und Z gleich O sind entweder
   a₁) zuerst mit Rhodanid zu einer Verbindung der Formel 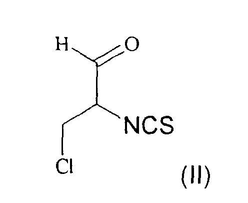 und anschließend mit einem Säure/R'OH- oder Säure/Orthoester- Gemisch, mit R' gleich C₁ bis C₆-Alkyl, zu der Verbindung der Formel 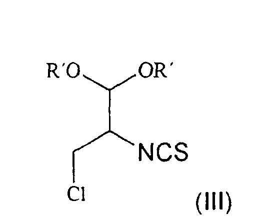 oder die Verbindung der Formel (I) zuerst in das Acetal überführt wird und dann mit Rhodanid zur Verbindung der Formel (III) umgesetzt wird, die sodann zu 2-Chlor-5-chlormethyl-1,3-thiazol umgesetzt wird
      oder
   a₂) mit Thioharnstoff zu einem Gemisch Verbindungen der Formeln 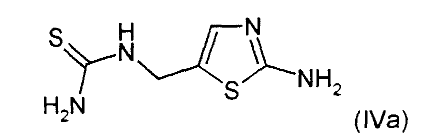 und 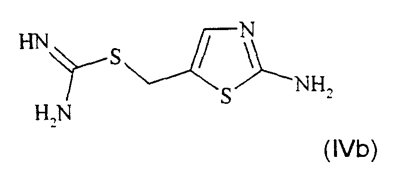 umgesetzt und anschließend nach basischer Spaltung zum entsprechenden Thiol bzw. Amin durch Sandmeyer-Diazotierung und gegebenenfalls Reaktion mit einem Chlorierungsmittel in 2-Chlor-5-chlormethyl-1,3-thiazol überführt wird
   oder
b) falls X gleich OR, SR oder NR₂, wobei R H oder eine geeignete Schutzgruppe bedeutet, Y gleich Cl und Z gleich O sind,
   mit Thioharnstoff zu einer Verbindung der Formel 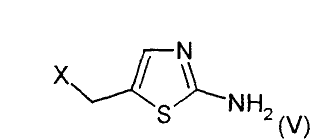 umgesetzt und anschließend durch Sandmeyer-Reaktion die Aminogruppe durch ein Chloratom ersetzt wird, worauf durch Chlorierung und gegebenenfalls Etherspaltung 2-Chlor-5-chlor-methyl -1,3-thiazol erhalten wird
   oder
c) falls X gleich OR, SR oder NR ₂ ist, wobei R H oder eine geeignete Schutzgruppe bedeutet, Y gleich Cl und Z gleich O sind,
   mit Ammoniumdithiocarbamat oder Ammoniumthiocarbamat zu der Verbindung 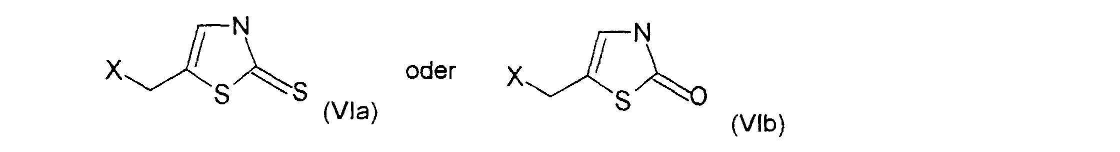 umgesetzt wird, worauf gegebenenfalls durch Abspaltung der Schutzgruppe der Rest X in den entsprechenden Rest OH, SH oder NH₂ überführt und anschließend über Reaktion mit geeigneten Chlorierungsmittein 2-Chlor-5-chlor-methyl -1,3-thiazol erhalten wird,
   oder
d) falls X, Y und Z gleich Cl sind, wobei die Verbindung keine Doppelbindung aufweist, mit Thioharnstoff zu einem Thiazolidin der Formel 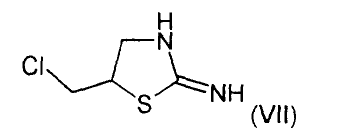 umgesetzt wird und anschließender Dehydrogenierung und Diazotierung zu 2-Chlor-5-chlormethyl-1,3-thiazol
   oder
e) falls X und Z gleich Cl und Y gleich H sind, wobei die Verbindung eine Doppelbindung zwischen C* und C" aufweist,
   durch Reaktion mit einem Oxidationsmittel in das entsprechende Epoxid überführt wird, das
   e₁) mit Thioharnstoff in einem geeigneten Lösungsmittel entweder direkt zu 2-Amino-5-chlormethyl-1,3-thiazol und/oder zu der Verbindung der Formel (V) mit X gleich OR', wobei R' H oder C₁-C₆-Alkyl bedeutet, umgesetzt wird und anschließend durch Diazotierung und gegebenenfalls Etherspaltung und/oder Chlorierung in 2-Chlor-5-chlormethyl-1,3-thiazol überführt wird oder
   e2) analog c) durch Umsetzung mit Ammoniumdithiocarbamat oder Ammoniumthiocarbamat zu 2-Chlor-5-chlormethyl-1,3-thiazol umgesetzt wird.

Als Ausgangsverbindung zur Herstellung von CCT wird erfindungsgemäß von einer Verbindung der Formel (I) ausgegangen, in der in der X Cl, -OR, -SR oder NR₂ bedeutet, wobei R H oder eine Schutzgruppe ist; Y H oder Cl und Z Cl oder O bedeuten, wobei die Verbindungen der Formel (I) maximal eine Doppelbindung zwischen C* und C" oder zwischen C" und Z aufweist, mit der Maßgabe, dass die Bindung zwischen C" und Z, wenn Z gleich O ist, eine Doppelbindung und wenn Z gleich Cl ist, eine Einfachbindung ist.
In der Formel (I) bedeutet der Rest R H oder eine Schutzgruppe. Als Schutzgruppe kommen alle Gruppen in Frage, die sich zum Schutz von des Sauerstoff-, Schwefel- oder Stickstoffrestes eignen. Dies sind unter anderem C₁-C₆-Alkylgruppen, wie etwa Methyl, Ethyl, Propyl, i-Butyl, t-Butyl, Hexyl, oder die Phthalimidgruppe.

### Variante a):

Bei Variante a) bedeuten X und Y Chlor und Z Sauerstoff, sodass als Ausgangsverbindung der Formel (I) 2,3-Dichlorpropanal eingesetzt wird.
2,3-Dichlorpropanal ist leicht zugänglich, beispielsweise durch Chlorierung von Acrolein in Dichlormethan.
Die Umsetzung des Aldehyds zu CCT kann erfindungsgemäß durch die Varianten a₁), oder a₂) erfolgen.

### Variante a₁):

Bei Variante a₁) kann der Aldehyd zuerst mit Na- oder NH₄-Rhodanid zu der Verbindung der Formel (II) umgesetzt werden. Das Rhodanid kann dabei sowohl äquimolar als auch im Über- oder im Unterschuss bezogen auf den Aldehyd eingesetzt werden. Bevorzugt wird das Rhodanid jedoch im Unterschuss eingesetzt. Die Umsetzung erfolgt dabei in einem geeigneten Lösungsmittel. Als Lösungsmittel kommen übliche organische Lösungsmittel in Frage. Dies sind beispielsweise Carbonsäuren mit 1 bis 6 C-Atomen, wie etwa in Ameisensäure, Essigsäure, Propionsäure u.s.w; halogenierte aliphatische und aromatische Kohlenwasserstoffe, wie etwa Methylenchlorid, Trichlormethan, Trichlorethylen, Tetrachlorkohlenstoff, Chlorbenzol, Dichlorbenzol u.s.w.; Alkohole, wie etwa Methanol, Ethanol, Propanol, t-Butanol, u.s.w.; Ether, wie etwa Diethylether, Dipropylether, Diisopropylether, Dibutylether, t.-Butyl-methylether, Ethylenglycolmonomethylether, Tetrahydrofuran, Dioxan u.s.w.; Ketone, wie etwa Aceton, Methyl-ethylketon, Methyl-i-butylketon, Cyclohexanon, u.s.w.; Amide, wie etwa N,N-Dimethylformamid, N,N-Diethylformamid N-Methylpyrrolidon, u.s.w.; Sulfoxide, wie etwa Dimethylsulfoxid, u.s.w. und Nitrile, wie etwa Acetonitril,Propionnitril, u.s.w.. Weiters eignen sich Wasser oder Lösungsmittel/Wasser-Gemische.
Gegebenenfalls kann es auch von Vorteil sein, dem Lösungsmittel ein Phasentransferkatalysator zuzusetzen. Die bevorzugte Menge an zugesetztem Phasentransferkatalysator beträgt dabei 0,1-15 mol%. Als Phasentransferkatalysator eignen sich beispielsweise Kronenether, quaternäre Ammoniumsalze, wie etwa Tetramethylammoniumchlorid, Tetraethylammoniumchlorid, Tetrabutylammoniumchlorid, Benzyltrimethylammoniumchlorid, sowie quaternäre Phosphoniumsalze.

Bevorzugte Lösungsmittel der Variante a₁) sind C₁-C₃-Carbonsäuren, Nitrile, chlorierte aliphatische Kohlenwasserstoffe und Amide. Besonders bevorzugt sind Essigsäure, Acetonitril, Dimethylformamid und ein Methylenchlorid/Kronenether - Gemisch.

Die Temperatur liegt zwischen 10 und 150 °C, bevorzugt zwischen 15 und 130°C, besonders bevorzugt zwischen 20 und 80 °C.

Die Verbindung der Formel (II) ist neu und deshalb ebenfalls Gegenstand der Erfindung.

Die Verbindung der Formel (II) wird sodann durch Zugabe eines Säure/R'OH- oder Säure/Orthoester- Gemisches bei Temperaturen von 10 bis 100°C in das Acetal der Formel (III) überführt.
Als Säuren eignen sich beispielsweise HCI oder p-Toluolsulfonsäure
Als Alkohol R'OH kommen C₁-C₆ Alkohole, wie Methanol, Ethanol, Propanol in Frage. Bevorzugt wird Ethanol eingesetzt.
Geeignete Orthoester sind beispielsweise Orthoameisensäurealkylester, wie etwa Orthoameisensäuremethyl- oder -ethylester.

Die Verbindung der Formel (III) ist neu und deshalb ebenfalls Gegenstand der Erfindung.

Der Aldehyd kann jedoch auch zuerst in das entsprechende Acetal überführt werden. Diese Überführung erfolgt analog obigen Verfahren. Anschließend wird das Acetal durch Reaktion mit Na- oder NH₄-Rhodanid zu der Verbindung der Formel (III) wiederum analog obigen Verfahren umgesetzt.

Die Verbindung der Formel (III) wird sodann durch geeignete Schritte, wie etwa Umlagerung, Abspaltung des Alkohols, bzw. Etherspaltung, Reaktion mit einem Chlorierungsmittel u.s.w. in 2-Chlor-5-chlormethyl-1,3-thiazol überführt.

Es ist weiters möglich, den Aldehyd der Formel (II) direkt durch geeignete Schritte in 2-Chlor-5-chlormethyl-1,3-thiazol zu überführen.

### Variante a₂):

Bei Variante a₂) wird der Aldehyd der Formel (I) zuerst mit Thioharnstoff zu einem Gemisch der Verbindungen N-[[5-(2-Aminothiazol)yl]methyl]thiourea und [5-(2-Aminothiazol)yl]methylthioformamidin der Formeln (IVa) und (IVb) umgesetzt, die in Form ihrer Hydrochloridsalze anfallen. Bevorzugt werden dabei 0,8 bis 2 Äquivalente an Thioharnstoff eingesetzt. Die Umsetzung erfolgt in einem der unter Variante a₁) angeführten geeigneten Lösungsmittel. Bevorzugt werden Ketone, wie etwa Methyl-i-Butyl-Keton, Aceton oder Alkohole, wie etwa Methanol, Ethanol, Butanol verwendet.
Die Reaktionstemperatur liegt zwischen 15°C und dem Siedepunkt des verwendeten Lösungsmittel.

Die Verbindungen der Formeln (IVa) und (IVb) bzw. deren Hydrochloridsalze sind ebenfalls neu und deshalb ein weiterer Gegenstand der Erfindung.

Die Umsetzung zu CCT erfolgt durch basische Spaltung zum entsprechenden Thiol bzw.Amin, anschließender Sandmeyer-Diazotierung und gegebenenfalls Chlorierung. Die Sandmeyer-Diazotierung erfolgt unter den für diese Reaktion üblichen Reaktionsbedingungen beispielsweise mit anorganischen oder organischen Nitriten, bevorzugt mit Natriumnitrit oder t-Butylnitrit in HCI (z.B. wässrige HCl) oder in Mischungen aus HCI und einem organischen polaren Lösungsmittel, wie etwa Acetonitril, gegebenenfalls in Anwesenheit eines Kupfer-Halogenid-Katalysators.

Geeignete Chlorierungsmittel sind solche Verbindungen, die unter den Reaktionsbedingungen chlorabspaltend wirken. Dies sind beispielsweise Cl₂, Sulfurylchlorid, PCl₅, PCl₃, POCl₃ u.s.w.
Die Reaktionsdurchführung bei der Chlorierung erfolgt in allgemein üblicher Art und Weise.

### Variante b):

Bei Variante b) bedeuten X einen Rest -OR, -SR oder NR₂ mit R gleich H oder geeignete Schutzgruppe; Y Cl und Z Sauerstoff.
Der entsprechende Aldehyd wird mit Thioharnstoff zu der Verbindung der Formel (V) umgesetzt. Thioharnstoff wird dabei in einer äquimolaren Menge, oder im Über- oder Unterschuss eingesetzt. Bevorzugt wird Thioharnstoff im leichten Unterschuss zum Aldeyd eingesetzt. Anschließend wird durch eine Sandmeyerreaktion analog Variante a₂) die Aminogruppe gegen ein Chloratom ausgetauscht.
Um CCT zu erhalten wird sodann gegebenenfalls die Ethergruppe gespalten und der entsprechende Rest durch ein Chloratom substituiert. Die Durchführung der Spaltung und Chlorierung erfolgt ebenfalls analog a₂).

### Variante c):

Bei Variante c) bedeutet X OR, SR oder NR₂ mit R gleich H oder geeignete Schutzgruppe, Y ist Cl und Z ist O.
Die entsprechende Verbindung der Formel (I) wird entweder mit Ammoniumdithiocarbamat oder mit Ammoniumthiocarbamat zu der Verbindung der Formel (Vla) bzw. (Vlb) umgesetzt.
Als Lösungsmittel kommen wiederum die unter Variante a1) angeführten Lösungsmittel in Frage. Bevorzugt werden Amide, Alkohole oder Nitrile verwendet. Besonders bevorzugt erfolgt die Reaktion in DMF, Methanol oder Ethanol, oder in Acetonitril.

Die Reaktionstemperatur liegt zwischen 0°C und dem Siedepunkt des verwendeten Lösungsmittel.
Anschließen wird gegebenenfalls der Rest X durch geeignete bekannte Methoden zur Schutzgruppenabspaltung gespalten, wodurch der jeweils entsprechende Rest OH, SH oder NH₂ erhalten wird. Je nach Schutzgruppe erfolgt die Abspaltung beispielsweise unter sauren, basischen oder hydrogenolytischen Bedingungen.
Die weitere Umsetzung zu CCT erfolgt durch Reaktion mit geeigneten Chlorierungsmitteln analog Variante a₂)

### Variante d)

Bei Variante d) bedeuten X, Y und Z ein Chloratom und die Verbindung weist keine Doppelbindung auf. Die Ausgangsverbindung ist demnach 1,2,3- Trichlorpropan, das mit Thioharnstoff zu dem Thiazolidin der Formel (VII) umgesetzt wird. Wiederum kann Thioharnstoff in äquimolarer Menge, im Unter- oder im Überschuss bezogen auf Trichlorpropan eingesetzt werden. Geeignete Lösungsmittel sind die unter Variante a₁) angeführten Lösungsmittel. Die Reaktionstemperaturen liegen zwischen 0°C und dem Siedepunkt des verwendeten Lösungsmittel.
Das Thiazolidin wird sodann durch Zugabe gebräuchlicher Oxidationsmittel zur Aromatisierung (üblicher Dehydrierungsmittel), wie etwa Schwefel, Chloranil, DDQ, Platinoxid u.s.w., unter den für Dehydrogenierungen üblichen Reaktionsbedingungen dehydogeniert.
Abschließend erfolgt die Sandmeyer-Diazotierung zu CCT bei für Diazotierungsreaktionen üblichen Reaktionsbedingungen analog Variante a₂).

### Variante e):

Bei Variante e) bedeuten X und Z ein Chloratom, Y ist H und die Verbindung weist eine Doppelbindung zwischen C* und C" auf. Als Ausgangsverbindung wird demnach 1,3-Dichlor-prop-1-en verwendet, das mit einem geeigneten Oxidationsmittel, wie etwa einer Peroxysäure, einem Säure/H₂O₂-Gemisch, anorganischen oder organischen Peroxiden oder Hydroperoxiden in 2-Chlor-3-chlormethyl-oxiran bei literaturbekannten Bedingungen, überführt wird.
Als Lösungsmittel eignen sich bevorzugt Nitrile, wie Acetonitril, oder chlorierte Kohlenwasserstoffe, wie etwa Chloroform, Tetrachlorkohlenstoff, Methylenchlorid 1,2-Dichlorethan oder Chlorbenzol.
Geeignete Peroxysäuren sind beispielsweise Peressigsäure, m-Chlor-perbenzoesäure, u.s.w.

Das erhaltene Oxiran bzw. Epoxid wird anschließend gemäß Variante e₁) analog den anderen Varianten mit Thioharnstoff in einem geeigneten Lösungsmittel zu der Verbindung der Formel (V) umgesetzt. Geeignete Lösungsmittel sind dabei die unter Variante a₁) angeführten Lösungsmittel. Bevorzugte Lösungsmittel der Variante e₁) sind Alkohole, insbesondere Methanol, Ethanol und t-Butanol, Ketone, insbesondere Aceton, Nitrile, insbesondere Acetonitril, Ether, insbesondere Tetrahydrofuran, Dimethoxyethan, Amide, insbesondere N-Methylpyrrolidon, Wasser oder Gemische mit Wasser, insbesondere Aceton/H₂O- oder Acetonitril/H₂O-Gemische. Es können auch Gemische eines Alkohols mit anderen Lösungsmittel, beispielsweise mit Dichlormethan eingesetzt werden. Bevorzugt sind deshalb auch Lösungsmittelgemische aus Alkohol, insbesondere Methanol, und Dichlormethan. Im Falle eines Lösungsmittelsgemisches kann es von Vorteil sein, wenn nachträglich eine geeignete Base, wie etwa ein Trialkylamin, zugesetzt wird.
Je nach Wahl des Lösungsmittels entsteht entweder direkt 2-Amino-5-chlormethyl-1,3-thiazol oder zuerst die Verbindung der Formel (V) mit R gleich R' oder aber Gemische aus 2-Amino-5-chlormethyl-1,3-thiazol und 2-Amino-5-Alkoxymethyl-1,3-thiazol und/oder 2-Amino-5-hydroxymethyl-1,3-thiazol. Die Verbindung der Formel (V) mit X gleich OR' entsteht insbesondere bei der Verwendung eines Alkohols als Lösungsmittel.
Die durch die Umsetzung mit Thioharnstoff erhaltene Verbindung oder das Gemisch obiger Verbindungen wird sodann durch Diazotierung und gegebenenfalls Etherspaltung und/oder Chlorierung in CCT überführt. Die Reihenfolge dieser Schritte kann dabei variieren.
Analog den anderen Varianten erfolgt durch Diazotierung der Austausch der Aminogruppe gegen ein Chloratom.
Wird im ersten Schritt die Verbindung der Formel (V) mit X gleich OR' oder ein oben beschriebenes Gemisch erhalten, so muss, um CCT zu erhalten, analog den bereits beschriebenen Varianten gegebenenfalls die Ethergruppe gespalten und eine Chlorierung durchgeführt werden. Die Etherspaltung, sowie die Chlorierung kann dabei vor, anschließend an die Diazotierung oder teilweise auch gleichzeitig mit der Diazotierung erfolgen.
Es ist auch möglich, die Etherspaltung und die Chlorierung in einem Schritt durchzuführen. Bevorzugt wird dabei durch Reaktion mit POCl₃ oder mit Acetylchlorid, gegebenenfalls in Kombination mit einer Lewis-Säure, wie etwa ZnCl₂, AlCl₃ oder BCl₃, oder mit trokkener HCI in Kombination mit einer Lewis-Säure, wie etwa ZnCl₂, AlCl₃ oder BCl₃, die C₁-C₆-Alkoxy-Gruppe des Restes OR' direkt, d.h. in einem Schritt, durch Cl ersetzt.
Eine andere Möglichkeit als die bisher beschriebenen Chlorierungsschritte um die C₁-C₆-Alkoxy-Gruppe oder die Hydroxy-Gruppe des Restes OR' durch ein Chloratom zu ersetzen, ist die Umsetzung des entsprechenden Thiazols mit Thionylchlorid.

Gemäß Variante e₂) wird das erhaltene Oxiran bzw. Epoxid analog zu Variante c) mit Ammoniumdithiocarbamat oder Ammoniumthiocarbamat zu CCT umgesetzt, wobei gegebenenfalls analog Variante e₁) die Spaltung des Restes X entfällt.

Die Isolierung und Aufarbeitung des hergestellten CCT erfolgt in Abhängigkeit von der gewählten Herstellungsvariante durch übliche Methoden, wie etwa Extraktion, Destillation u.s.w.

### Beispiel 1: Herstellung eines Gemisches aus N-[[5-(2-Aminothiazol)yl]methyl]thiourea und [5-(2-Amino-thiazol)yl]methylthioformamidin

4,8g (37,8 mMol) 2,3-Dichlorpropionaldehyd wurden mit 2,80g (36,8 mMol) Thioharnstoff in 25ml absolutem Ethanol für 3 Stunden zum Sieden erhitzt. Der entstandene Feststoff wurde abfiltriert, heiß mit Ethanol und anschließend zweimal mit Aceton digeriert.
Ausbeute: 2,05g (42% d. Th.)
Analysen:
^{***1***}***H-NMR*** (DMSO-d₆): δ(ppm): 9,70 (s; 1H; breit); 9,63 (s; 2H, breit); 9,43 (s; 2H, breit); 7,43 (s; 1H); 4,81 (s; 2H)
^{***13***}***C-NMR*** (DMSO-d₆): δ (ppm): 170,7 (s ); 169,3 (s); 125,3 (d); 120,1 (s); 28,6 (t) ***Elementaranalyse***: berechnet: C: 22,99%; H: 3,86; N: 21,54; Cl: 27,15%; S: 24,55%
gefunden: C: 22,9%; H: 3,9%; N: 21,4%; Cl: 27,1%; S: 24,5%

### Beispiel 2: 3-Chlor-2-isothiocyanatopropanal

### Methode A

36,35g (0,29 Mol) 2,3-Dichlorpropionaldehyd und 23,51g (0,29 Mol) Nartiumthiocyanat wurden in 250ml Eisessig für 2 Stunden auf 50°C erhitzt. Das Lösungsmittel wurde abgezogen, der Rückstand in 500ml Methylenchlorid aufgenommen und der entstandene Niederschlag abfiltriert. Das Filtrat wurde zweimal mit je 200ml Wasser gewaschen, über Natriumsulfat getrocknet und das Lösungsmittel abgezogen.
Ausbeute: 32,10g 3-Chlor-2-isothiocyanatopropanal; orange-gelbes Öl (74% d.Th.)

### Methode B

6,35 g (50 mMol) 2,3-Dichlorpropionaldehyd und 2,43 g (30 mMol) Natriumthiocyanat wurden in 15 ml Acetonitril 3 Stunden zum Sieden erhitzt. Die Suspension wurde filtriert und das Filtrat vom Lösungsmittel befreit. Der Rückstand wurde in 50 ml Methylenchlorid gelöst, unlösliche Anteile wurden abfiltriert und das Filtrat zweimal mit je 10 ml Wasser gewaschen und die Produktlösung durch azeotrope Destillation getrocknet.
Ausbeute: entspricht laut GC der theoretischen Ausbeute
Analysen:
***Kp***: 60-62°C /1mbar
^{***1***}***H-NMR*** (CDCl₃): 9,58 (s; 1H); 4,56 (t; 1H; J = 5,5 Hz); 4,11 (d; 2H; J = 5,5 Hz)
^{***13***}***C-NMR*** (CDCl₃): 192,9 (d); 134,3 (s, sehr klein); 60,3 (d); 47,5 (t)

### Beispiel 3: 3-Chlor-1,1-diethoxy-2-isothiocyanatopropan

6,75 g (45 mMol) 3-Chlor-2-isothiocyanatopropionaldehyd wurden in 67 ml Ethanol gelöst, mit 67 ml konzentrierter Salzsäure versetzt und für 2 Stunden zum Sieden erhitzt. Die Reaktionslösung wurde 4 mal mit je 100ml Methylenchlorid extrahiert, die vereinten organischen Phasen mit Wasser und Natriumhydrogencarbonat-Lösung gewaschen und anschließend das Lösungsmittel abgezogen, worauf 3-Chlor-1,1-diethoxy-2-isothiocyanatopropan erhalten wurde.
Analysen:
^{**1**}**H-NMR** (CDCl₃): δ(ppm): 4,59 (d; 1H; J = 5,2 Hz); 4,01-3,92 (m; 1H); 3,91 (t; 2H; J = 5,7 Hz); 3,82 - 3,70 (m, 2H); 3,68 - 3,56 (m; 2H); 1,29 - 1,18 (m; 6H)
^{**13**}**C-NMR** (CDCl₃): δ(ppm): 102,6 (d); 66,9 (t); 64,3 (t); 59,8 (d); 48,0 (t); 15,5 (q)
**GC/MS**: m/z: 103, 47, 75, 29, 72, 27, 150, 178, 59, 142, 152, 180

### Beispiel 4: 2-Chlor-5-methoxymethylthiazol

Zu 2,54g (20,0 mMol) 1,3-Dichlorpropenoxid in 6 ml Methanol wurde bei 2°C eine Lösung von 1,29g (17 mMol) Thioharnstoff in 15 ml Methanol zugetropft und zunächst eine Stunde bei Raumtemperatur und dann eine weitere Stunde bei Siedetemperatur gerührt.
Das Lösungsmittel wurde abgezogen, der Rückstand zwischen Natriumhydrogencarbonat-Lösung und tert-Butyl-Methyl-Ether verteilt und das Zwischenprodukt erschöpfend extrahiert.
Das Zwischenprodukt (1,40g) wurde in 5 ml Salzsäure und 1,6 ml Acetonitril gelöst und bei 2°C mit 0,9g (9,4 mMol) Kupfer-(I)-chlorid versetzt, danach wurde bei -8°C bis 0°C eine Lösung von 1,51g (13,2 mMol) tert-Butylnitrit in 1,6 ml Acetonitril langsam zugetropft. Danach wurde noch für 2 Stunden bei 0-5°C und eine weitere Stunde bei Raumtemperatur gerührt.
Das Reaktionsgemisch wurde filtriert und dann erschöpfend mit Chloroform extrahiert. Die organischen Phasen wurden mit Natriumhydrogencarbonat-Lösung und Wasser gewaschen und vom Lösungsmittel befreit.
Ausbeute: 1,31g 2-Chlor-5-methoxymethylthiazol; gelbes Öl (40% d.Th.)
Analysen:
¹H-NMR (CDCl₃): δ(ppm): 7,41 (s; 1H); 4,56 (s; 2H); 3,40 (s; 3H)
GC-MS: m/z: 163, 128, 72, 57

### Beispiel 5: Weiterverarbeitung von 2-Chlor-5-methoxymethylthiazol zu CCT

### Methode A)

0,1g (0,61 mMol) 2-Chlor-5-methoxymethylthiazol, hergestellt nach Beispiel 4, wurden in einem Gemisch von 1ml Wasser, 1ml Schwefelsäure und 0,5 ml Salzsäure 5 Stunden bei Raumtemperatur und weitere 5 Stunden bei 50°C gerührt. Die Reaktionslösung wurde mit Toluol extrahiert und der Extrakt mittels GC-MS analysiert. Dabei konnte mit Hilfe von Vergleichsspektren eindeutig CCT als Endprodukt identifiziert werden.
GC/MS : m/z: 128, 132, 45, 163, 134, 71, 29, 72, 57, 58, 39, 79, 165

### Methode B)

2,00g (12,22 mMol) 2-Chlor-5-methoxymethylthiazol, hergestellt nach Beispiel 4, wurden mit 4,00g (26,20 mMol) Phosphoroxychlorid für 12 Stunden auf 80°C erhitzt. Danach wurde das Reaktionsgemisch auf Eiswasser gegossen und 3 mal mit Methylenchlorid extrahiert. Der Extrakt enthielt als Hauptprodukt CCT, das mit Hilfe von Vergleichsspektren eindeutig identifiziert wurde. Nebenprodukte waren mittels GC nicht zu erkennen.

### Beispiel 6: 2-Chlor-5-methoxymethylthiazol

Zu 7,62g (60,0 mMol) 1,3-Dichlorpropenoxid in 18 ml Methanol wurde bei 2°C eine Lösung von 3,65g (48 mMol) Thioharnstoff in 50 ml Methanol zugetropft und anschließend eine Stunde bei Siedetemperatur gerührt.
Das Lösungsmittel wurde abgezogen, das Zwischenprodukt (10,1g) in 25 ml Salzsäure und 8 ml Acetonitril gelöst und bei 2°C mit 4,75g (48 mMol) Kupfer-(I)-chlorid versetzt, danach wurde bei -10°C bis -5°C eine Lösung von 6,93g (67,2 mMol) tert-Butylnitrit in 8,2 ml Acetonitril langsam zugetropft. Danach wurde noch für 3 Stunden bei Raumtemperatur gerührt.
Das Reaktionsgemisch wurde filtriert und dann erschöpfend mit Chloroform extrahiert. Die organischen Phasen wurden mit Natriumhydrogencarbonat-Lösung und Wasser gewaschen und vom Lösungsmittel befreit.
Ausbeute; 7,05g 2-Chlor-5-methoxymethylthiazol; gelbes Öl (90% d.Th.)
Analysen:
¹H-NMR (CDCl₃): δ(ppm): 7,41 (s; 1H); 4,56 (s; 2H); 3,40 (s; 3H)
GC-MS: m/z: 163, 128, 72, 57

### Beispiel 7: Weiterverarbeitung von 2-Chlor-5-methoxymethylthiazol zu CCT

0,1g (0,61 mMol) 2-Chlor-5-methoxymethylthiazol, hergestellt nach Beispiel 6, wurden in einem Gemisch von 1ml Wasser, 1ml Schwefelsäure und 0,5 ml Salzsäure 5 Stunden bei Raumtemperatur und weitere 5 Stunden bei 50°C gerührt. Die Reaktionslösung wurde mit Toluol extrahiert und der Extrakt mittels GC-MS analysiert. Dabei konnte mit Hilfe von Vergleichsspektren eindeutig CCT als Endprodukt identifiziert werden.

### Beispiel 8:

98,8g (0,89 Mol) 1,3-Dichlorpropen wurden in 400g 1,2-Dichlorethan gelöst und mit 155,6g (0,9 Mol) getrockneter 3-Chlorperbenzoesäure versetzt und für 40 Stunden auf 60°C erhitzt. Das Reaktionsgemisch wurde auf -5°C gekühlt, die 3-Chlorbenzoesäure abfiltriert und das Filtrat mit gesättigter Natriumhydrogencarbonatlösung und 10%iger Natriumpyrosulfit-Lösung gewaschen. Die wäßrigen Phasen wurden einmal mit Chloroform extrahiert und anschließend die vereinten organischen Phasen über Natriumsulfat getrocknet, filtriert und das Lösungsmittel abgezogen. Zuletzt wird das Produkt bei 80-83°C /60 mbar destilliert.
Ausbeute: 84,75g 1,3-Dichlorpropenoxid; farblose Flüssigkeit (83%d.Th.; Gehalt 88%) Anschließend wurden 1,29g (17,0 mMol) Thioharnstoff und 2,54g (17,0 mMol) 88%iges 1,3-Dichlorpropenoxid in 21 ml 1,2-Dimethoxyethan für 2 Stunden auf 60°C erhitzt und danach das Lösungsmittel abgezogen.
Das rohe 2-Amino-5-chlormethylthiazol wurde in 10 ml konzentrierter Salzsäure und 3,4ml Acetonitril suspendiert und bei -13 bis -7°C zunächst mit 1,98 g (20 mMol) Kupfer(I)-chlorid, dann langsam mit 2,89 g (28 mMol) tert. Butylnitrit in 3,4 ml Acetonitril versetzt und 2 Stunden bei Raumtemperatur gerührt.
Die Reaktionslösung wurde mit 20 ml Wasser verdünnt, erschöpfend mit Dichlormethan extrahiert und zuletzt das Lösungsmittel abgezogen. Dieses Rohprodukt, welches neben 37Gew.% CCT noch 2-Chlorhydroxymethylthiazol enthielt, wurde mit 1 ml Thionylchlorid versetzt und eine Stunde bei Raumtemperatur gerührt. Die Reaktionslödung wurde mit 25 g Eis hydrolysiert, erschöpfend mit Dichlormethan extrahiert und zuletzt das Lösungsmittel abgezogen.
Ausbeute: 2,42 g CCT,; braunes Öl (Gehalt 52Gew%; 44% d.Th.)

## Patentansprüche

1. Verfahren zur Herstellung von 2-Chlor-5-chlormethyl-1,3-thiazol, **dadurch gekennzeichnet, dass** eine Verbindung der Formel 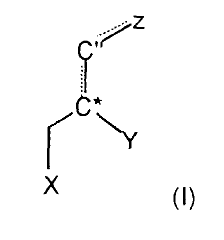 in der X Cl, -OR, -SR oder NR₂ bedeutet, wobei R H oder eine geeignete Schutzgruppe ist; Y H oder Cl und Z Cl oder O bedeuten,
wobei die Verbindungen der Formel (I) maximal eine Doppelbindung zwischen C* und C" oder zwischen C" und Z aufweist, mit der Maßgabe, dass die Bindung zwischen C" und Z, wenn Z gleich O ist, eine Doppelbindung und wenn Z gleich Cl ist, eine Einfachbindung
ist,
a) falls X und Y gleich Cl und Z gleich O sind
entweder
a₁) zuerst mit Rhodanid zu einer Verbindung der Formel 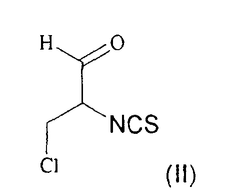 und anschließend mit einem Säure/R'OH- oder Säure/Orthoester- Gemisch, mit R' gleich C₁ bis C₆-Alkyl, zu der Verbindung der Formel 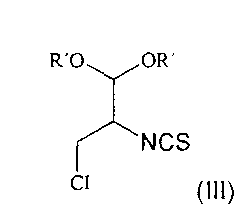 oder die Verbindung der Formel (I) zuerst in das Acetal überführt wird und dann mit Rhodanid zur Verbindung der Formel (III) umgesetzt wird, die sodann zu 2-Chlor-5-chlormethyl-1,3-thiazol umgesetzt wird
oder
a₂) mit Thioharnstoff zu einem Gemisch Verbindungen der Formeln 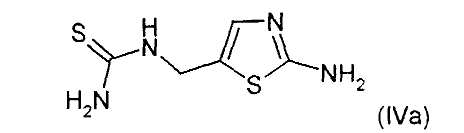 und 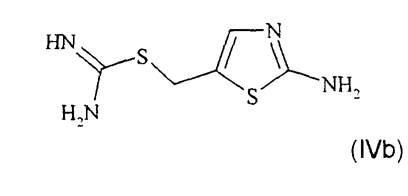 umgesetzt und anschließend nach basischer Spaltung zum entsprechenden Thiol bzw. Amin durch Sandmeyer-Diazotierung und gegebenenfalls Reaktion mit einem Chlorierungsmittel in 2-Chlor-5-chlormethyl-1,3-thiazol überführt wird
oder
b) falls X gleich OR, SR oder NR₂, wobei R H oder eine geeignete Schutzgruppe bedeutet, Y gleich Cl und Z gleich O sind,
mit Thioharnstoff zu einer Verbindung der Formel 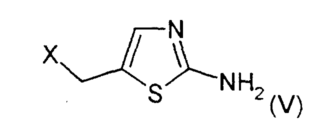 umgesetzt und anschließend durch Sandmeyer-Reaktion die Aminogruppe durch ein Chloratom ersetzt wird, worauf durch Chlorierung und gegebenenfalls Etherspaltung 2-Chlor-5-chlor-methyl -1,3-thiazol erhalten wird
oder
c) falls X gleich OR, SR oder NR ₂ ist, wobei R H oder eine geeignete Schutzgruppe bedeutet, Y gleich Cl und Z gleich O sind
mit Ammoniumdithiocarbamat oder Ammoniumthiocarbamat zu der Verbindung 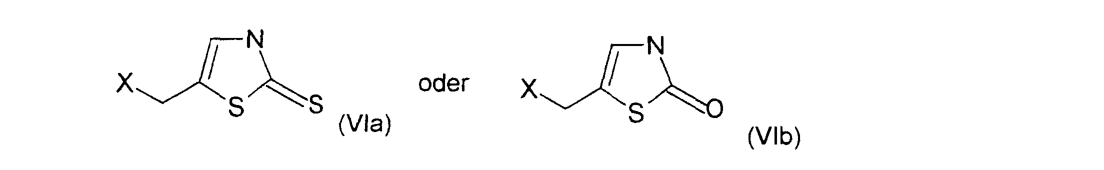 umgesetzt wird, worauf gegebenenfalls durch Abspaltung der Schutzgruppe der Rest X in den entsprechenden Rest OH, SH oder NH₂ überführt und anschließend über Reaktion mit geeigneten Chlorierungsmitteln 2-Chlor-5-chlor-methyl -1,3-thiazol erhalten wird,
oder
d) falls X, Y und Z gleich Cl sind, wobei die Verbindung keine Doppelbindung aufweist, die Verbindung der Formel (I) mit Thioharnstoff zu einem Thiazolidin der Formel 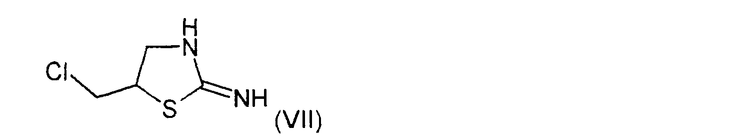 umgesetzt wird und anschließender Dehydrogenierung und Diazotierung zu 2-Chlor-5-chlormethyl-1,3-thiazol
oder
e) falls X und Z gleich Cl und Y gleich H sind, wobei die Verbindung eine Doppelbindung zwischen C* und C" aufweist,
durch Reaktion mit einem Oxidationsmittel in das entsprechende Epoxid überführt wird, das
e₁) mit Thioharnstoff in einem geeigneten Lösungsmittel entweder direkt zu 2-Amino-5-chlormethyl-1,3-thiazol und/oder zu der Verbindung der Formel (V) mit X gleich OR', wobei R' H oder C₁-C₆-Alkyl bedeutet, umgesetzt wird und anschließend durch Diazotierung und gegebenenfalls Etherspaltung und/oder Chlorierung in 2-Chlor-5-chlormethyl-1,3-thiazol überführt wird oder
e2) analog c) durch Umsetzung mit Ammoniumdithiocarbamat oder Ammoniumthiocarbamat zu 2-Chlor-5-chlormethyl-1,3-thiazol umgesetzt wird.

2. Verfahren nach Anspruch 1, **dadurch gekennzeichnet, dass** gemäß Variante a₁) 2,3-Dichlorpropanal entweder zuerst mit Na- oder NH₄-Rhodanid in einem organischen Lösungsmittel, gegebenenfalls in Anwesenheit eines Phasentransferkatalysators zu 3-Chlor-2-isothiocyanatopropanal und anschließend zu 3-Chlor-1,1-dialkoxy-2-isothiocyanatopropan umgesetzt wird oder zuerst die Umwandlung in das entsprechende Acetal des 2,3-Dichlorpropanals erfolgt und anschließend die Reaktion mit Na- oder NH₄-Rhodanid zu 3-Chlor-1,1-dialkoxy-2-isothiocyanatopropan erfolgt.

3. Verfahren nach Anspruch 1, **dadurch gekennzeichnet, dass** gemäß Variante a₂) 2,3-Dichlorpropanal mit 0,8 bis 2 Äquivalenten an Thioharnstoff zu einem Gemisch der Verbindungen der Formeln (IVa) und (IVb) umgesetzt wird, wobei die Verbindungen der Formeln (IVa) und (IVb) in Form eines Hydrochloridsalzes anfallen.

4. Verfahren nach Anspruch 1, **dadurch gekennzeichnet, dass** gemäß Variante d) 1,2,3-Trichlorpropan mit Thioharnstoff zu dem Thiazolidin der Formel (VII) umgesetzt wird, das durch Zugabe eines Oxidationsmittels dehydrogeniert und anschließend diazotiert wird.

5. Verfahren nach Anspruch 1, **dadurch gekennzeichnet, dass** gemäß Variante e₁) die Etherspaltung und Chlorierung in einem Schritt erfolgt, wobei die C₁-C₆-Alkoxy-Gruppe der Verbindungen der Formel (V) durch Reaktion mit POCl₃ oder mit Acetylchlorid, gegebenenfalls in Kombination mit einer Lewis-Säure, oder mit trockener HCl in Kombination mit einer Lewis-Säure direkt durch ein Chloratom substituiert wird.

6. Verfahren nach Anspruch 1, **dadurch gekennzeichnet, dass** gemäß Variante e₁) die C₁-C₆-Alkoxy-Gruppe oder die Hydroxygruppe der Verbindungen der Formel (V) durch Reaktion mit Thionylchlorid durch Chlor substituiert wird.

7. Eine Verbindung der Formel (II), wie in Anspruch 1 definiert.

8. Eine Verbindung der Formel (III), wie in Anspruch 1 definiert.

9. Verbindungen der Formel (IVa) und (IVb), wie in Anspruch 1 definiert und deren Hydrochloridsalze.

10. Verwendung der Verbindungen gemäß den Ansprüchen 7, 8 and 9 bei der Herstellung von 2-Chlor-5-chlormethyl-1,3-thiazol.

## Claims

1. A process for the preparation of 2-chloro-5-chloromethyl-1,3-thiazole, which comprises reacting a compound of the formula 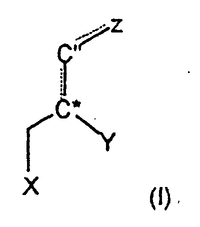 in which X is Cl, -OR, -SR or -NR₂, where R is H or a suitable protecting group; Y is H or Cl; and Z is Cl or O,
the compounds of the formula (I) having a maximum of one double bond between C* and C" or between C" and Z, with the proviso that the bond between C" and Z is a double bond when Z is O and is a single bond when Z is Cl,
a) where X and Y are Cl and is O
either
a₁) first with thiocyanate to give a compound of the formula 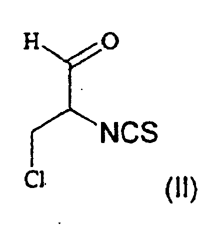 and then with an acid/R'OH or acid/orthoester mixture, where R' is C₁ to C₆-alkyl to give the compound of the formula 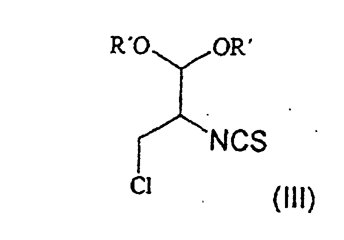 or first converting the compound of the formula (I) to the acetal and then reacting with thiocyanate to give the compound of the formula (III), then converting it to 2-chloro-5-chloromethyl-1,3-thiazole,
or
a₂) with thiourea to give a mixture of compounds of the formulae 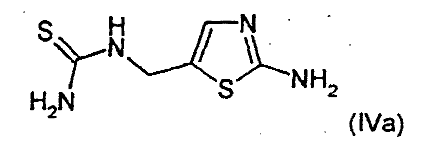 and 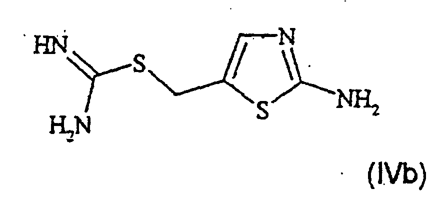 and, after basic cleavage to give the corresponding thiol or amine, converting to 2-chloro-5-chloromethyl-1,3-thiazole by Sandmeyer diazotization and optional reaction with a chlorinating agent
or
b) where X is OR, SR or NR₂, and R is H or a suitable protecting group, Y is Cl and Z is O,
with thiourea to give a compound of the formula 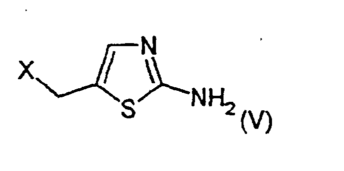 and then substituting the amino group by a chlorine atom by Sandmeyer reaction, and obtaining 2-chloro-5-chloromethyl-1,3-thiazole by chlorination and optional ether cleavage
or
c) where X is OR, SR or NR₂, and R is H or a suitable protecting group, Y is Cl and Z is O,
with ammonium dithiocarbamate or ammonium thiocarbamate to give the compound 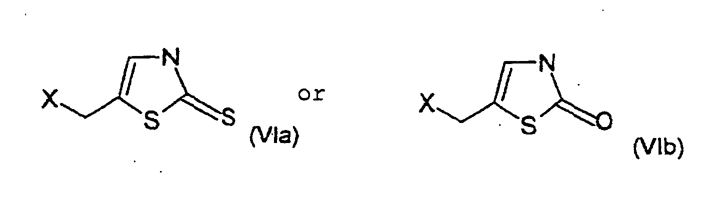 and, if necessary, converting the radical X into the corresponding radical OH, SH or NH₂ by removal of the protecting group, and then obtaining 2-chloro-5-chloromethyl-1,3-thiazole by reaction with suitable chlorinating agents,
or
d) where X, Y and Z are all Cl, and the compound contains no double bonds,
the compound of the formula (I) with thiourea to give a thiazolidine of the formula 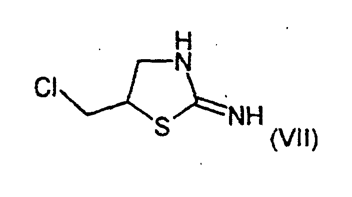 and then dehydrogenating and diazotizing to give 2-chloro-5-chloromethyl-1,3-thiazole
or
e) where X and Z are both Cl and Y is H, and the compound contains a double bond between C* and C", with an oxidizing agent to convert it to the corresponding epoxide, which
e₁) is converted directly to 2-amino-5-chloromethyl-1,3-thiazole using thiourea in a suitable solvent and/or is converted to the compound of the formula (V), where X is OR', and R' is H or C₁-C₆-alkyl, and is then converted to 2-chloro-5-chloromethyl-1,3-thiazole by diazotization and, if necessary, ether cleavage and/or chlorination, or
e₂) similarly to c), is converted to 2-chloro-5-chloromethyl-1,3-thiazole by reaction with ammonium dithiocarbamate or ammonium thiocarbamate.

2. The process as claimed in claim 1, wherein, in. variant a₁), 2,3-dichloropropanal is either first converted to 3-chloro-2-isothiocyanatopropanal by sodium or ammonium thiocyanate in an organic solvent, and optionally in the presence of a phase transfer catalyst, and then to 3-chloro-1,1-dialkoxy-2-isothiocyanatopropane, or is first converted to the corresponding acetal of 2,3-dichloropropanal before reaction with sodium or ammonium thiocyanate to give 3-chloro-1,1-dialkoxy-2-isothiocyanatopropane.

3. The process as claimed in claim 1, wherein, in variant a₂), 2,3-dichloropropanal is reacted with from 0.8 to 2 equivalents of thiourea to give a mixture of compounds of the formulae (IVa) and (IVb) which occur in the form of a hydrochloride salt.

4. The process as claimed in claim 1, wherein, in variant d), 1,2,3-trichloropropane is reacted with thiourea to give the thiazolidine of the formula (VII), which is dehydrogenated by addition of an oxidizing agent and then diazotized.

5. The process as claimed in claim 1, wherein, in variant e₁), the ether cleavage and chlorination take place in a single step which involves substituting the C₁-C₆-alkoxyl group of the compounds of the formula (V) directly by a chlorine atom by reaction with POCl₃ or an acetyl chloride, optionally in combination with a Lewis acid, or with dry HCl in combination with a Lewis acid.

6. The process as claimed in claim 1, wherein, in variant e₁), the C₁-C₆-alkoxyl group or the hydroxyl group of the compounds of the formula (V) is substituted by chlorine by reaction with thionyl chloride.

7. A compound of the formula (II), as defined in claim 1.

8. A compound of the formula (III), as defined in claim 1.

9. Compounds of the formulae (IVa) and (IVb), as defined in claim 1, and their hydrochloride salts.

10. Use of the compounds as claimed in claims 7, 8 and 9 in the preparation of 2-chloro-5-chloromethyl-1,3-thiazole.

## Revendications

1. Procédé de préparation de 2-chloro-5-chlorométhyl-1,3-thiazol **caractérisé en ce qu'**un composé de formule 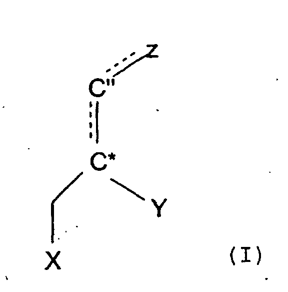 dans laquelle X représente Cl, -OR, -SR ou NR₂, R signifiant H ou un groupe protecteur approprié ; Y. signifiant H ou Cl et Z Cl ou O,
les composés de formule (I) présentant au maximum une double liaison entre C* et C" ou entre C" et Z, à la condition que la liaison entre C" et Z, quand Z est égal à O, soit une double liaison et, quand Z est égal à Cl, soit une simple liaison,
a) si X et Y sont égaux à Cl et Z est égal à O
soit
a₁) est transformé d'abord avec le rhodanide en un composé de formule 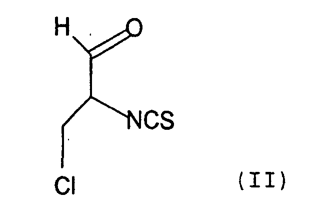 et ensuite avec un mélange acide/R'OH- ou acide/orthoester, avec R' égal à alkyle en C₁ à C₆, en un composé de formule 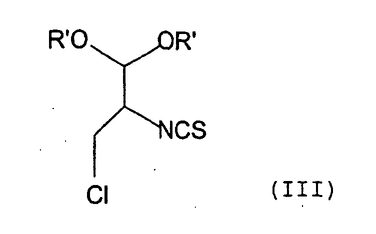 ou le composé de formule (I) est d'abord transformé en acétal et ensuite converti avec du rhodanide en composé de formule (III) qui est ensuite converti en 2-chloro-5-chlorométhyl-1,3-thiazol
ou
a₂) est converti avec de la thiourée en un mélange de composés de formules 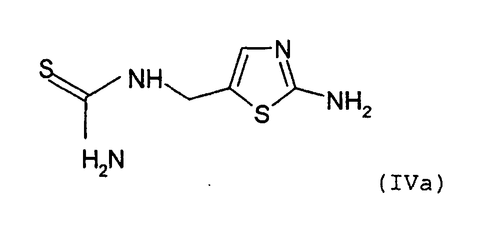 et 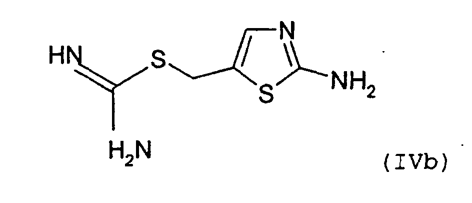 et ensuite transformé, après séparation basique en le thiol correspondant ou l'amine correspondante par diazotation de Sandmeyer et éventuellement réaction avec un agent de chloration, en 2-chloro-5-chlorométhyl-1,3-thiazol
ou
b) si X est égal à OR, SR ou NR₂, R signifiant H ou un groupe protecteur approprié, Y est égal à Cl et Z à O,
est converti avec de la thiourée en un composé de formule 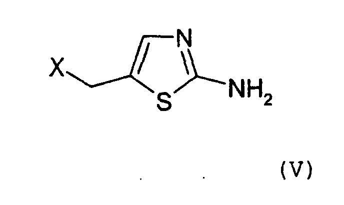 et ensuite, par réaction de Sandmeyer, le groupe amine est substitué par un atome de chlore, après quoi on obtient, par chloration et éventuellement séparation à l'éther, du 2-chloro-5-chlorométhyl-1,3-thiazol
ou c) si X est égal à OR, SR ou NR₂, R signifiant H ou un groupe protecteur approprié, Y est égal à Cl et Z à O,
est converti avec du dithiocarbamate d'ammonium ou du thiocarbamate d'ammonium en le composé 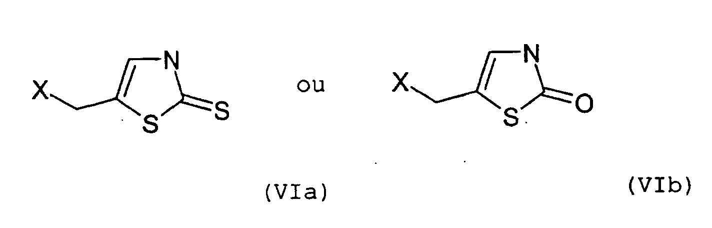 après quoi on obtient éventuellement par élimination du groupe protecteur, le radical X est transformé en le radical correspondant OH, SH ou NH₂ et ensuite, le 2-chloro-5-chlorométhyl-1,3-thiazol est obtenu par réaction avec les agents de chloration appropriés,
ou d) si X, Y et Z sont égaux à Cl, le composé ne présentant aucune double liaison,
est converti avec de la thiourée en une thiazolidine de formule 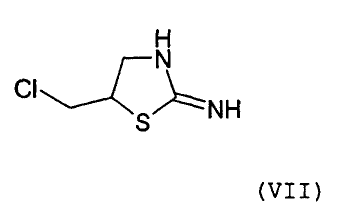 et, par déshydrogénation et diazotation subséquentes, en 2-chloro-5-chlorométhyl-1,3-thiazol
ou
e) si X et Z sont égaux à Cl et Y est égal à H, le composé présentant une double liaison entre C* et C",
est transformé par réaction avec un agent d'oxydation en l'époxyde correspondant qui e₁) est converti avec de la thiourée dans un solvant approprié soit directement en 2-chloro-5-chlorométhyl-1,3-thiazol et/ou en le composé de formule (V) avec X égal OR', R' signifiant H ou alkyle en C₁-C₆, et ensuite, est transformé, par diazotation et éventuellement séparation de l'éther et/ou chloration, en 2-chloro-5-chlorométhyl-1,3-thiazol ou
e₂) de manière analogue à c) est converti, par conversion avec du dithiocarbamate d'ammonium ou thiocarbamate d'ammonium en 2-chloro-5-chlorométhyl-1,3-thiazol.

2. Procédé selon la revendication 1, **caractérisé en ce que** selon la variante a₁), le 2,3-dichloropropanal est converti soit d'abord avec du rhodanide Na ou NH₄ dans un solvant organique, éventuellement en présence d'un catalyseur de transfert de phases en 3-chloro-2-isothiocyanatopropanal et ensuite en 3-chloro-1,1-dialcoxy-2-isothiocyanatopropane soit on effectue d'abord la conversion en l'acétal correspondant du 2,3-dichloropropanal et ensuite la réaction avec le rhodanide de Na ou de NH₄ en 3-chloro-1,1-dialcoxy-2-isothiocyanatopropane.

3. Procédé selon la revendication 1, **caractérisé en ce que** selon la variante a₂) le 2,3-dichloropropanal est converti avec 0,8 à 2 équivalents de thiourée en un mélange de composés de formules (IVa) et (IVb), les composés de formules (IVa) et (IVb) se présentant sous forme de sels chlorhydriques.

4. Procédé selon la revendication 1, **caractérisé en ce que** selon la variante d) le 1,2,3-trichloropropane est converti avec la thiourée en thiazolidine de formule (VII), qui est déshydrogénée par addition d'un agent d'oxydation et ensuite diazotée.

5. Procédé selon la revendication 1, **caractérisé en ce que** selon la variante e₁) la séparation de l'éther et la chloration s'effectuent en une étape, le groupe alcoxy en C₁-C₆ des composés de formule (V) étant substitué directement par un atome de chlore, par réaction avec du POCl₃ ou avec du chlorure d'acétyle, éventuellement en combinaison avec un acide de Lewis ou avec du HCl sec en combinaison avec un acide de Lewis.

6. Procédé selon la revendication 1, **caractérisé en ce que** selon la variante e₁) le groupe alcoxy en C₁-C₆ ou le groupe hydroxy des composés de formule (V) est substitué par le chlore par réaction avec du chlorure de thionyle.

7. Composé de formule (II), tel que défini dans la revendication 1.

8. Composé de formule (III), tel que défini dans la revendication 1.

9. Composés de formule (IVa) et (IVb), tels que définis dans la revendication 1, et leurs sels chlorhydriques.

10. Utilisation des composés selon les revendications 7, 8 et 9, lors de la préparation du 2-chloro-5-chlorométhyl-1,3-thiazol.
